(19) Europäisches Patentamt
European Patent Office
Office européen des brevets

(11) **EP 4 645 338 A1**

(12) **EUROPEAN PATENT APPLICATION**

(43) Date of publication:
**05.11.2025 Bulletin 2025/45**

(21) Application number: **25174093.2**

(22) Date of filing: **02.05.2025**

(51) International Patent Classification (IPC):
**G16H 50/30** (2018.01)     **G16H 50/20** (2018.01)
**G16H 30/40** (2018.01)     **G16H 50/70** (2018.01)
**G06T 7/00** (2017.01)

(52) Cooperative Patent Classification (CPC):
**G16H 50/30; G06N 3/045; G06N 3/0455;
G06N 3/0464; G06N 3/048; G06N 3/08;
G06N 3/084; G06N 3/09; G06N 5/01; G06T 7/0012;
G16H 30/40; G16H 50/20; G16H 50/70;**
G06T 2207/10056; G06T 2207/20081;     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **03.05.2024 GB 202406267**

(71) Applicant: **Oxford Cancer Biomarkers Ltd
Oxford OX4 4GA (GB)**

(72) Inventors:
• **XU, Xianhong
Oxford (GB)**
• **KERR, David
Oxford (GB)**

(74) Representative: **Elkington and Fife LLP
Prospect House
8 Pembroke Road
Sevenoaks, Kent TN13 1XR (GB)**

(54) **HISTOPATHOLOGY-BASED SOLID TUMOUR ANALYSIS**

(57)     A computer-implemented method is provided, for predicting a risk factor for a patient based on histopathological image analysis. The method includes: receiving (110) at least one histological image of a solid tumour; converting (150) the histological image into a graph representation; processing (160) the graph representation using a neural network, wherein the neural network comprises a graph isomorphism network and a convolutional neural network; and determining (162) the risk factor based on an output of the neural network. Also provided is a method of training one or more neural networks for use in such a method.

FIG. 1

(52) Cooperative Patent Classification (CPC): (Cont.)
G06T 2207/20084; G06T 2207/30024;
G06T 2207/30096

**Description**

FIELD

The present disclosure relates to image analysis. In particular, it relates to analysis of histological images.

BACKGROUND

Approximately 90 percent of adult cancers are solid tumours. A resection surgery is the most common method for the treatment of early-stage solid tumour patients. However, the necessity and the efficacy of post-surgery treatments such as adjuvant chemotherapy for early-stage cancers is often uncertain due to the heterogeneity of the disease. Stage II colorectal cancer (CRC) patients, in particular, have a relatively high 5-year overall survival rate after surgical resection alone (approximately 80%). The remaining 20% consists of 4% of patients who would benefit from adjuvant chemotherapy and 16% who may not be susceptible to adjuvant chemotherapy. There is a clinical need for predictive markers to risk-stratify patients so that there is a better understanding of those at a greater risk of recurrence. This would delineate clearer and more personalised treatment pathways for patients within this group, minimising over-treatment as the toxicity associated with adjuvant chemotherapy may pose significant challenges for individual patients.

However, developing such a prognosis marker for early-stage cancers represents a significant challenge. In the current clinical pathway, clinicians rely on traditional indicators and factors to estimate the risk of the disease. Some of these indicators include TNM stage, invasion and margin status, number of lymph nodes, DNA mismatch repair (MMR) status, and microsatellite instability (MSI) condition. The assessment of these indicators and the estimation of prognosis rely on the clinical expertise and judgment of the healthcare professionals involved.

SUMMARY

A computer-implemented method is provided, for predicting a risk factor for a patient based on histopathological image analysis. The method includes: receiving at least one histological image of a solid tumour; converting the histological image into a graph representation; processing the graph representation using a neural network, wherein the neural network comprises a graph isomorphism network and a convolutional neural network; and determining the risk factor based on an output of the neural network. Also provided is a method of training one or more neural networks for use in such a method.

According to one aspect, there is provided a computer-implemented method for predicting a risk factor for a patient based on histopathological image analysis, the method comprising:

   receiving at least one histological image showing a sample of a solid tumour;
   converting the histological image into a graph representation;
   processing the graph representation using a neural network, wherein the neural network comprises a graph isomorphism network and a convolutional neural network; and
   determining the risk factor based on an output of the neural network.

The risk factor may indicate one of a plurality of levels of risk - for example, high-risk, intermediate risk and low risk. The risk factor may indicate a risk of relapse of a disease (optionally cancer, for example colorectal cancer).

The risk factor may be determined using the Cox proportional hazards model.

The convolutional neural network (CNN) may be a one-dimensional (1D) convolutional neural network (1D-CNN). The method may comprise providing concatenated node presentation data from the graph representation as an input to the 1D-CNN.

The histological image may be of a tissue slide, optionally a stained tissue slide, optionally a hematoxylin and eosin (H&E) stained tissue slide.

Optionally, the neural network is a first neural network; the output of the first neural network comprises a risk index prediction; and the method comprises: processing the graph representation using a second neural network; and determining the risk factor based on the risk index prediction and an output of the second neural network.

The risk index prediction may be a numerical value, optionally a real number. Thus, the risk index prediction may comprise a continuous one-dimensional variable.

The second neural network may comprise a second graph isomorphism network and a second convolutional neural network.

The second convolutional neural network may be a 1D-CNN.

Each graph isomorphism network (GIN) may comprise one or more GIN convolutional layers and a global pooling layer. Each GIN convolutional layer consists of a stack of multilayer perceptron (MLP) networks and a learnable scalar value that represents the importance of a node compared to its neighbours. A plurality of sub-graphs may be extracted from the graph representation by selecting a node and its immediate neighbours (connected by graph edges to the selected node). When processing each such sub-graph in the GIN, the scalar value acts as a kind of weight "added" to the central node of the neighbourhood. However, the term "added" should not be understood as limiting to a linear summation - the "weighting" operation may be nonlinear. In general, the scalar value may be fixed or may be a learnable parameter (via machine learning during the training phase).

Each convolutional neural network (optionally, a 1D-CNN) may comprise: one or more convolutional layers, and optionally one or more pooling layers, and further optionally at least one fully connected layer.

The output of the second neural network may comprise a prognosis class prediction.

The prognosis class prediction may be a binary class prediction. For example, it may be a binary classification indicating whether the patient belongs to a good prognosis group or not.

The risk index prediction may be a prediction of a cancer specific death risk index.

Determining the risk factor may comprise combining the risk index prediction with the output of the second neural network.

In particular, this may comprise combining the risk index prediction with the prognosis class prediction.

The risk factor may be determined to be "high" responsive to the risk index prediction exceeding a first threshold. The risk factor may be determined to be "intermediate" responsive to the risk index prediction being less than or equal to the first threshold and greater than a second threshold (wherein the second threshold is lower than the first threshold). The risk factor may be determined to be "low" responsive to the risk index prediction being less than or equal to the second threshold and the prognosis class prediction indicating that the patient has a "good" prognosis (that is, indicating that the patient belongs to the good prognosis group). The risk factor may be determined to be "intermediate" responsive to the risk index prediction being less than or equal to the second threshold and the prognosis class prediction indicating that the patient does not have a "good" prognosis.

The neural network may receive as an input one or more clinical parameters of the patient, and the output of the neural network may be based at least in part on the one or more clinical parameters.

In some examples, the one or more clinical parameters may be input to a layer of the convolutional neural network (hereinafter, CNN). In some examples, the one or more clinical parameters are input to a fully connected layer of the CNN.

The clinical parameters may comprise any one, or any combination of two or more, of: an age of the patient; a sex of the patient; an ethnicity of the patient; a tumour stage or other estimate of the condition of a tumour; a lymph-node stage or other estimate of the condition of the lymph-node; and a tumour site. The tumour stage and lymph-node stage may be obtained from a pathologist. The tumour site may indicate which part of an organ is affected by a tumour. For example, in the case of colorectal cancer, the tumour site may be indicated as one of the following categories: left, right, or top.

The second neural network may also receive as input one or more clinical parameters of the patient, and the output of the second neural network may be based at least in part on the one or more clinical parameters. The one or more clinical parameters input to the second neural network may be the same as or different from the one or more clinical parameters input to the first neural network.

The one or more clinical parameters may be input to a layer of the second CNN In some examples, the one or more clinical parameters are input to a fully connected layer of the second CNN.

Converting the histological image into the graph representation may comprise dividing at least a part of the image into blocks of pixels and constructing the graph representation based on the blocks. The blocks may be nonoverlapping.

Each block may be represented by a respective node in the graph. Two nodes may be linked by an edge in the graph based at least in part on a spatial distance between them. The method may comprise extracting a plurality of features from each block, wherein two nodes may be linked by an edge based at least in part on a similarity between their extracted features.

The method may comprise extracting a region of interest from the histological image and dividing the region of interest, in particular, into the blocks of pixels. Further details relating to the region of interest extraction will be provided below.

Converting the histological image into the graph representation may comprise extracting a plurality of features from each of at least some of the blocks, wherein the extracting optionally comprises applying the respective block as input to a neural network configured in a self-distillation with no labels, hereinafter DINO, architecture.

The DINO architecture may comprise a pair of encoder neural network models. The encoder neural network models may comprise a student encoder and a teacher encoder. Each encoder may comprise a vision transformer (ViT) neural network.

Converting the histological image into a graph representation optionally comprises extracting a region of interest from the histological image and constructing the graph representation based on the region of interest.

Extracting the region of interest may comprise generating a mask image defining the region of interest.

Extracting the region of interest may comprise applying a first machine learning model a first scale and applying a second machine learning model at a second scale.

The at least one histological image may comprise a first image at a first magnification (for example 5x) and a second image at a second magnification (for example, 20x). The first machine learning model may be applied to the first image. An output of the first machine learning model may include a first mask image.

The second machine learning model may be applied to the second image. In some examples, the second machine learning model may be applied separately to each of a plurality of portions of the second image. An output of the second machine learning model may include a respective partial mask image for each of the plurality of portions of the second image. The method may comprise combining the partial mask images into a second mask image.

The method may comprise rescaling at least one of the first mask image and the second mask image (e.g. rescaling one to the same scale as the other). The method may comprise combining the (optionally rescaled) first mask image and the (optionally rescaled) second mask image into a combined mask image. Combining the mask images may comprise applying a logical OR function.

The method may comprise applying morphological processing operations to at least one of: the combined mask image, first mask image, the second mask image, and the partial mask images. The morphological processing operations may comprise at least one of: dilation, and closing, for example.

At least one, or each, of the first machine learning model and the second machine learning model may comprise a neural network, for example a CNN, for example a Deep CNN (DCNN).

Also provided is a computer implemented method of training a machine learning architecture for predicting a risk factor for a patient based on histopathological image analysis, the machine learning architecture comprising a neural network, the method comprising:

> obtaining a plurality of histological images;
> obtaining a patient outcome associated with each image;
> converting each histological image into a respective graph representation; and
> training the neural network to predict the risk factor using the graph representations and the respective patient outcomes,
> wherein the neural network comprises a graph isomorphism network and a convolutional neural network.

The neural network may be neural network as summarised above.

The neural network may be a first neural network, wherein the output of the first neural network comprises a risk index prediction. The machine learning architecture may further comprise a second neural network. The method may further comprise training the second neural network, wherein the second neural network is configured to process the graph representation and the machine learning architecture is configured to determine the risk factor based on the risk index prediction and an output of the second neural network.

Converting each histological image into the respective graph representation may comprise dividing at least a part of the image into blocks of pixels and constructing the graph representation based on the blocks.

Converting each histological image into the respective graph representation may comprise extracting a plurality of features from each block, comprising applying the block as input to a neural network configured in a self-distillation with no labels, hereinafter DINO, architecture.

The method may further comprise training the DINO architecture to extract the features, based on the plurality of histological images and the patient outcomes.

The patient may have a cancer, optionally colorectal cancer.

Also provided is a computer program comprising computer program code configured to cause one or more physical computing devices to perform a method as summarised above when said computer program code is run on the one or more physical computing devices. The computer program may be stored on a computer readable storage medium (optionally non-transitory).

Also provided is a system for predicting a risk factor for a patient based on histopathological image analysis, the system comprising:

> an input, for receiving at least one histological image showing a sample of a solid tumour; and
> one or more processors, configured to:

>> convert the histological image into a graph representation;
>> process the graph representation using a neural network, wherein the neural network comprises a graph isomorphism network and a convolutional neural network; and
>> determine the risk factor based on an output of the neural network.

The system may further comprise a scanner, for example a digital pathology scanner, configured to scan a tissue slide to generate the at least one histological image. The scanner may be coupled to the input and may be configured to communicate the scanned histological image via the input - for example, in the form of a whole slide image (WSI).

According to another aspect, there is disclosed a method of stratifying patients (for example, for different treatments), the method comprising:

for each of a plurality of patients, predicting a risk factor using a method as summarised above; and
stratifying the patients based on their respective predicted risk factors.

According to another aspect, there is disclosed, a method of treating a patient, the method comprising:

predicting a risk factor for the patient, using a method as summarised above;
selecting a treatment for the patient, based on the predicted risk factor; and
treating the patient according to the selected treatment.

The patient may be a cancer patient, optionally a colorectal cancer patient.

The treatment may comprise one or both of: surgery to resect a tumour; and chemotherapy (optionally combination chemotherapy, using two or more medicaments). The chemotherapy may be adjuvant chemotherapy.

For example, adjuvant chemotherapy may be selected responsive to the predicted risk factor indicating a high risk.

In some examples, selecting a treatment for the patient may comprise selecting not to treat the patient. For instance, the method may comprise selecting not to treat the patient responsive to the predicted risk factor indicating a low risk.

According to still another aspect, there is provided a pharmaceutical composition for use in treatment of a solid tumour, said treatment being indicated at least in part based on a risk factor predicted using a trained machine learning model from a histological image showing a sample of the solid tumour.

The solid tumour may be malignant. In particular, the solid tumour may be a colorectal cancer tumour.

The pharmaceutical composition may be for chemotherapy. The pharmaceutical composition may comprise a DNA synthesis inhibitor. The pharmaceutical composition may comprise: capecitabine, or bevacizumab, or a combination of capecitabine and bevacizumab.

The risk factor may be predicted using a method as summarised previously above.

BRIEF DESCRIPTION OF THE DRAWINGS

The invention will now be described by way of example with reference to the accompanying drawings, in which:

Fig. 1 is a flowchart illustrating a method according to an example;
Fig. 2 shows an example of an image of a H&E stained tissue slide;
Fig. 3 is a flowchart illustrating a method of extracting a region of interest, according to an example;
Fig. 4 illustrates an example of feature extraction using a DINO architecture;
Fig. 5 is a flowchart illustrating a method of predicting a prognosis risk category, according to an example;
Fig. 6 is a flowchart illustrating a GIN-based prediction, according to an example;
Fig. 7 illustrates algorithm for combining a predicted risk index value with a predicted prognosis class, according to an example;
Fig. 8 shows the results of experiments evaluating a method according to an example;
Fig. 9 is a flowchart illustrating a method of training a neural network, according to an example;
Fig. 10 is a block diagram showing an exemplary computer, by which methods according to examples of the present disclosure may be performed.

It should be noted that these figures are diagrammatic and not drawn to scale. Relative dimensions and proportions of parts of these figures have been shown exaggerated or reduced in size, for the sake of clarity and convenience in the drawings.

DETAILED DESCRIPTION

The tumour microenvironment (TME) can play an important role in predicting cancer recurrence. Tumour cells can instigate substantial molecular, cellular, and physical changes in the surrounding stroma. Immune cells, such as myeloid-derived suppressor cells (MDSCs), macrophages, and tumour-infiltrating lymphocytes (TILs) within the stroma, play a role in suppressing tumour growth and spread. Such alterations can be captured visually in haematoxylin and eosin (H&E)-stained images. Analysing these stained images can allow for the identification and interpretation of features related to the TME, contributing to a better understanding of the cancer, and aiding in the prediction of recurrence risk.

Examples according to the present disclosure use histopathology images of H&E stained tissue samples collected from the solid tumours of cancer patients to predict cancer relapse risk levels (known as prognosis) based on artificial intelligence technology. Examples according to the present disclosure can offer a precise and personalized prognosis for cancer patients, which may empower clinicians to make informed decisions regarding the tailored use of adjuvant chemotherapy for individual patients.

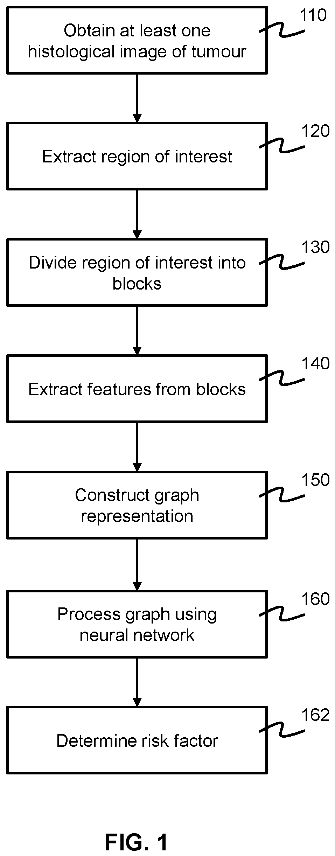

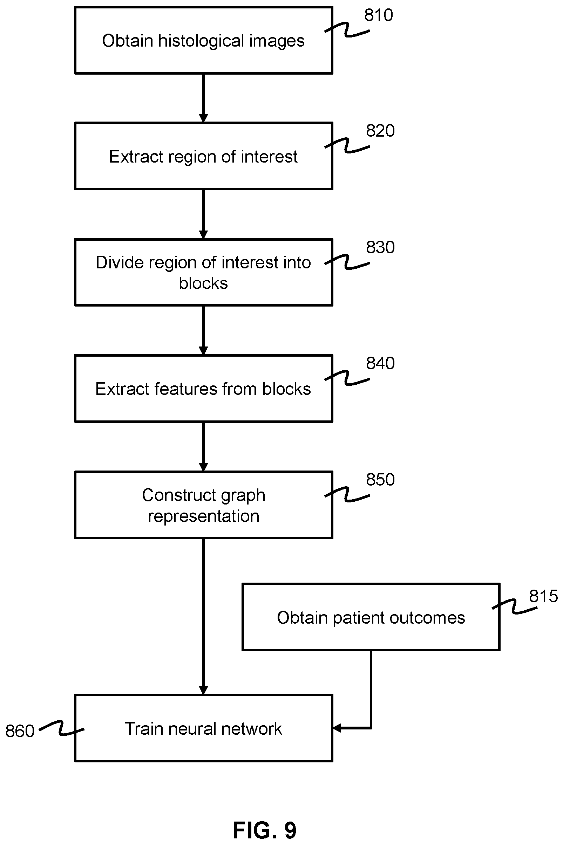

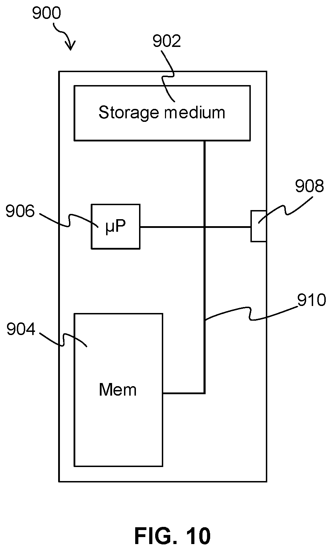

Fig. 1 is a flowchart illustrating a method of analysing a histological image to predict a risk factor for a patient, according to one example. The method may be implemented by a computer. (An exemplary computer will be described later below, with reference to Fig. 10.) It should be understood that the method of Fig. 1 relates to the inference phase. Machine learning models used in the inference phase can be trained offline, in advance. This preparatory stage is referred to as the training phase. In normal use, the images presented to the system in the inference phase are "new" images, not previously seen by the system in the training phase. That is, the images input in the inference phase are normally not images that were included in a training dataset. An exemplary training method will be described later, below, with reference to Fig. 9.

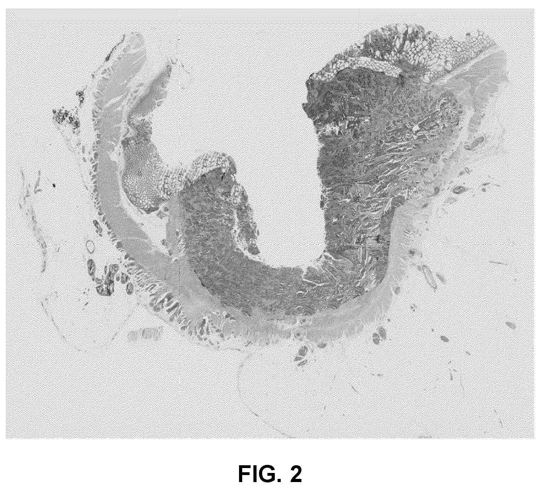

In operation 110, the computer obtains at least one histological image showing a sample of a solid tumour. In the present example, the histological image is a H&E stained image and the solid tumour is a malignant tumour, in particular, a colorectal cancer tumour. An example of a histological image of this type is shown in Fig. 2.

H&E staining is well known in the field of histopathology. In digital pathology, the slides prepared in the pathology laboratory are scanned using a digital pathology scanner into a whole slide image (WSI). A WSI image is a composite of red, green, blue (RGB) images of different scales in a pyramid. Each of the images in the pyramid can be characterized and identified by using the magnification of the object lens of the scanner, such as 5x, 10x, 20x, 40x. The maximum magnification of the images used in the present example is 40x.

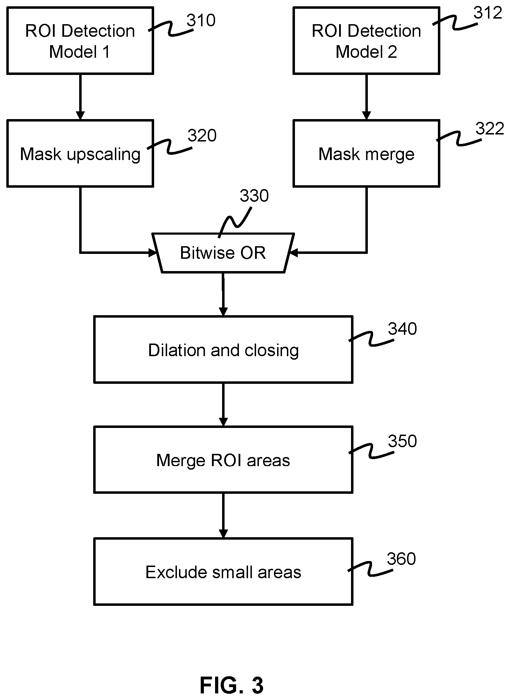

In operation 120, the computer extracts a region of interest from the histological image. This is essentially an image segmentation operation. The region of interest is the portion of the image that corresponds to the tumour. In the present example, the output of the operation 120 is a binary mask image indicating the region of interest. One exemplary algorithm for automatic detection of the region of interest will be discussed below, with reference to Fig. 3.

In operation 130, the computer divides the region of interest extracted in operation 120 into blocks (which may also be referred to as "tiles" or "tile images"). In the present example, this is based on the region of interest in the image with maximum magnification (that is, the 40x image). By way of example, this region of interest may be divided into 256 x 256 nonoverlapping blocks.

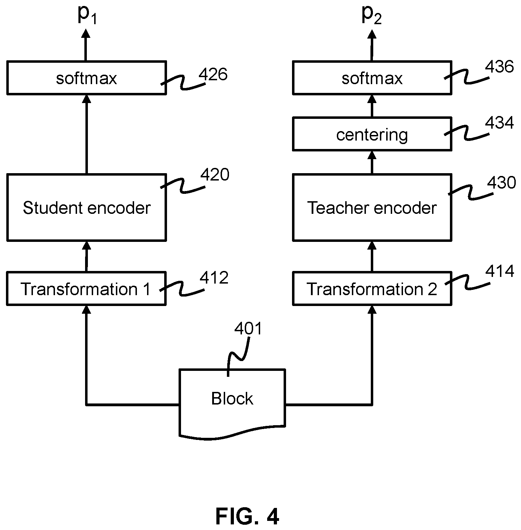

In operation 140, the computer extracts image features from each of the blocks obtained in operation 130. In the present example, the feature extraction is based on a self-supervised deep learning model. This will be described in more detail below, with reference to Fig. 4.

In operation 150, the computer constructs a graph representation of the histological image. It does this using the image features extracted (in operation 140) from the blocks in the region of interest.

In operation 160, the computer processes the graph representation constructed in operation 150, using a neural network. This neural network comprises a graph isomorphism network (GIN) and a convolutional neural network (CNN).

In operation 162, the computer uses the output of the neural network to determine a risk factor for the patient. In the present example, the risk factor is determined in the form of a predicted prognosis risk category. Three risk categories are defined: high, intermediate, and low. The computer classifies the patient into one of these predicted categories, based on the output of the neural network.

By way of example, the risk categories may be defined as follows:

- A cancer-specific death (CSD) within three years indicates the patient being in the high risk (poor prognosis) group;
- A disease-free survival (DFS) beyond five years indicates the patient being in the low risk (good prognosis) group;
- All other patients are in the intermediate risk (non-distinct prognosis) group.

While these definitions have been found to provide useful results, it should be understood that this example is nonlimiting. In other examples, the risk categories to be predicted may be defined differently. Alternatively, the risk factor that is determined based on the output of the neural network might not be in the form of a categorisation - it could be in the form of a probability or other scalar value.

Further details of the operations in the example of Fig. 1 will now be provided.

The flowchart of Fig. 3 illustrates one exemplary method for extracting a region of interest from a histological image (operation 120). The WSI is processed in this operation to identify the specific region that is most informative for assessing cancer recurrence risk. This is referred to as the region of interest (ROI). The ROI area can include tumour cells, the surrounding stroma, and the immune cells, such as myeloid-derived suppressor cells (MDSCs), macrophages, and tumour-infiltrating lymphocytes (TILs) within the stroma that play a role in suppressing tumour growth and spread.

The exemplary ROI detection image processing algorithm of Fig. 3 is based on a supervised deep learning neural network, DeepLabv3+, which was proposed by Chen et al. for semantic image segmentation (Liang-Chieh Chen, Yukun Zhu, George Papandreou, Florian Schroff, Hartwig Adam, "Encoder-Decoder with Atrous Separable Convolution for Semantic Image Segmentation", European Conference on Computer Vision 2018, pp 833-851). The neural network uses deep convolutional neural networks (DCNN) and spatial pyramid pooling and adopts an encoder-decoder structure.

In the method of Fig. 3, the histological image is processed at multiple resolutions. In operation 310, the 5x magnification image of the WSI is fed to a first region of interest (ROI) detection model. The output of this operation is a single global mask (at a low resolution). This mask is upsampled (upscaled) to a higher resolution in operation 320.

The 20x magnification image of the WSI is divided into 16 nonoverlapping, equal-size sub-images. Each of these sub-images is fed to a second ROI detection model, in operation 312. The second ROI detection model produces a mask (at the higher resolution) for each sub-image. These are merged (stitched) together into a single larger mask in operation 322.

The masks produced in operations 320 and 322 are combined in operation 330, using a logical bitwise OR operator. This produces a combined mask that contains the union of the regions detected by both models.

In the present example, the two ROI detection models used in operations 310 and 312 have the same network architecture. MobileNetV2 is used as the backbone module for image feature extraction.

To train the model, a dataset of 1100 H&E images and related pathological annotation data was collected. Based on the doctor's annotation of the lesion area, we obtained a dataset with lesion tissue markers and normal tissue markers. In the training process, random flipping, resizing, rotation, and H&E enhancement methods were performed to enhance the data so that the model would be more robust to image variability.

Before analysing the whole slide images, they were pre-processed (as part of operation 110) with a H&E normalisation algorithm (M Macenko, M Niethammer, JS Marron, D Borland, JT Woosley, G Xiaojun, C Schmitt, NE Thomas, "A method for normalizing histology slides for quantitative analysis", IEEE ISBI, 2009. dx.doi.org/10.1109/ISBI.2009.5193250) to compensate for lighting and/or staining conditions. The normalisation parameters were collected from the same data set of H&E images. All subsequent processing of the images was performed using the normalised versions.

The combined mask produced by operation 330 is subjected to postprocessing in operations 340, 350, and 360. Morphological filtering is performed in operation 340, in the form of dilation and closing. In operation 350, discrete (that is, non-contiguous) areas of the mask are merged if the shortest distance between them is less than a threshold distance (for example, 5 pixels in the 20x resolution). In operation 360, contiguous areas less than a predetermined minimum threshold area (number of pixels) are eliminated. The postprocessing can help to reduce noise in the mask. According to the presently described example, only the pixels in the detected region of interest will be processed further in analysis method. (These pixels are indicated by the active pixels in the final mask that is output from operation 360.)

As mentioned previously, the region of interest identified by the mask is divided into blocks in operation 130. In operation 140, feature extraction is applied to each block to produce image features characterising the content of that block.

Fig. 4 shows a flowchart illustrating an example of the feature extraction process. In this example, the TME information embedded in the H&E image is extracted using a self-supervised deep learning model - namely, the DINO (Self-distillation with **no** labels) architecture proposed by Caron et al. (Mathilde Caron, Hugo Touvron, Ishan Misra, Hervé Jégou, Julien Mairal, Piotr Bojanowski, Armand Joulin, "Emerging Properties in Self-Supervised Vision Transformers", May 2021, https://arxiv.org/abs/2104.14294.)

The feature extraction is run for each block (or "tile") separately. Fig. 4 shows one of these blocks 401 being provided as input to the feature extraction process. During the model-training process, a pair of encoder neural network models, namely, the "student" 420 and the "teacher" 430 are employed in the DINO architecture (as shown in Figure 4). The input block 401 is subjected to two different random transformations 412, 414 and the transformed versions are passed to the student and teacher networks, respectively. Both encoder networks 420, 430 have the same architecture but different parameters. The output of the teacher network is centred (see operation 434) with a mean (exponential moving average, EMA) computed over the training batch. Each encoder network 420, 430 outputs an N-dimensional feature that is normalized with a temperature SoftMax (see operations 426 and 436) over the feature dimension. The teacher parameters are updated with an exponential moving average (ema) of the student parameters.

In the present example, a Vision Transformer (ViT) is used as the encoder in the feature extraction model. ViT is a deep learning architecture specifically designed for image processing tasks and its superior performance is evident on several large public image datasets. The following parameters are used in the ViT encoder according to the present example:

- Depth, L = 12
- Number of heads = 6
- Number of features = 384

In the model training process, operations 120 and 130 are performed on each of the training images. This produces a set of blocks (tiles) from the region of interest in the 40x image of each training image. These blocks (tiles) are fed into the DINO-style feature extraction neural networks and the networks are trained.

All the whole slide images (WSIs) in the training dataset were used when training the self-supervised model, to maximise robustness to variation between the WSIs, as much as possible. In order to reduce the computational burden (and therefore calculation time), a Monte Carlo random selection algorithm was used to select 20% of tiles in each image, while making sure that the tiles selected adequately represent the corresponding WSI.

After training, the trained teacher encoder is saved and then is used, in the inference phase, in operation 140, to extract the features from the blocks (tiles) produced in operation 130.

In operation 150, a graph representation is constructed from the features extracted in operation 140. In the present example, the rules used to construct the graph are as follows:

- Each block is represented by a node in the graph.
- The spatial location of each block in the whole slide image represent defines the location of the respective node.
- Each node is linked to its (spatially) nearest neighbouring nodes by an edge in the graph, partly based on a comparison of the features extracted in operation 140, and partly based on a distance threshold. In particular, the adjacency matrix ($A_{ij}$) between nodes is determined as follows:

$$A_{ij} = \begin{cases} 1 \; if \; j \in KNN(i) \; \text{and} \; D(i,j) < d \\ 0 \; \text{otherwise} \end{cases}$$

Here, $KNN(i)$ is the set of K nearest neighbours of node i, in the feature space defined by the feature extraction in operation 140. (In the present example, K=11.) Meanwhile, $D(i,j)$ denotes the spatial distance between the centre of the block corresponding to node i and the centre of the block corresponding to node j. The distance threshold, d, is chosen empirically.

- The block feature data extracted in operation 140 is used as the embedding of each node.

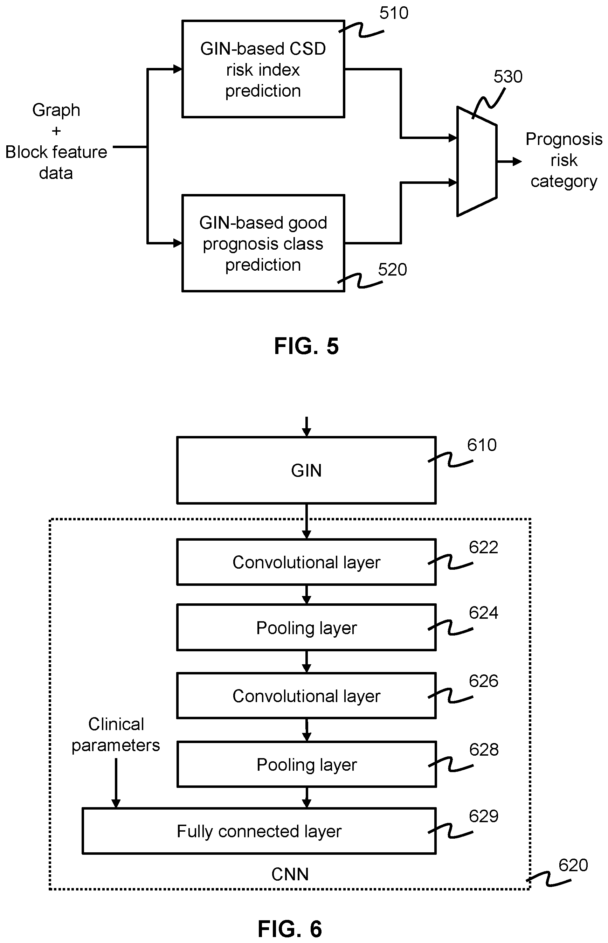

Fig. 5 is a flowchart illustrating a method of predicting a prognosis risk category, according to an example. The input data for this method consists of the graph constructed in operation 150 (along with the block feature data, which provide the node-embeddings). In the method, the input data is processed using two neural networks. Both are based on a graph isomorphic network (GIN) architecture combined with a convolutional neural network (CNN). A first GIN-based neural network 510 is trained to predict a cancer specific death (CSD) risk index. A second GIN-based neural network 520 is trained to predict whether the patient belongs to the good prognosis (low risk) class. The outputs of the two neural networks 510 and 520 are combined in operation 530, to produce a prediction of the prognosis risk category.

The CSD risk index prediction model 510 and the good prognosis class prediction model 520 share the same GIN+CNN architecture. This is shown in Fig. 6.

The graph (constructed in operation 150) is input to the GIN model (see operation 610). In the present example, the GIN model uses node embeddings, sum pooling, and concatenating as the global pooling (graph-level readout) for producing a graph embedding. However, this is not limiting. For instance, other types of pooling could be used - including, but not limited to, mean or max pooling.

As the graph generated from the WSI contains many nodes (blocks), a follow-on convolutional neural network 620 is used for extracting the graph features. This design allows the effective use of the graph data (i.e. the feature of the tile images) for prognosis analysis. The CNN is a 1D-CNN operating on the concatenated graph readout from the GIN 610. In the present example, the CNN comprises (in the following order): a first convolutional layer 622; a first pooling layer 624; a second convolutional layer 626; and a second pooling layer 628.

The output of the second pooling layer 628 is provided as an input to a fully connected layer 629. In addition, selected clinical parameters are encoded as high-level embeddings to feed into the fully-connected layer 629 of the CNN 620. In the present example, these are: patient age; patient gender; patient ethnicity; pT stage; pN stage; and tumour site. The "pT stage" refers to the tumour stage, for example as judged by a pathologist. The "pN stage" refers to a lymph-node stage, for example as judged by a pathologist. The tumour site is indicated as "left", "right", or "top". (It should be understood that this classification of the tumour site was designed in the context of colorectal cancer - other classifications may be appropriate for other cancers/organs.)

It has been found that the combination of the graph isomorphism network followed by a (1D) convolutional network produces good results, in terms of predicting both the CSD index and the prognosis class. It has further been found that the inclusion of clinical parameters among the inputs to the CNN can boost the prediction accuracy.

The neural network model for the CSD risk index prediction 510 will now be described in more detail. This model is used to conduct a lifetime analysis on the WSI graph. We use cox proportional hazards models (CPH) to evaluate cancer specific survival (CSS) time. A general hazard function $\lambda(t/x)$, which represents the risk of dying at time $t$ can be estimated as follows:

$$\lambda(t|x) = \lambda_0(t)e^{h(x)}$$

This is a semi-parametric survival prediction model. In the formula above:

- $t$ represents survival time.
- $\lambda_0(t)$ represents a baseline hazard function. The variable t indicates it is a time dependent value.
- $h(x)$ is the log-risk function, which is a linear function related to the patient. The $h(x)$ power of e ($e^{h(x)}$) is the hazard ratio. x represents a vector of the clinical covariates observed.

The baseline hazard function does not need to be specified for the CPH model, which is concerned with h(x) only and is therefore semi-parametric. We defined a cancer specific death risk index (CSDI) function based on the h(x) as follows:

$$CSDI(x) = \frac{1}{1 + e^{-\alpha \cdot h(x) + \beta}}$$

Here, $\alpha$ and $\beta$ are real numbers. This establishes a direct relationship between the CSDI(x) function and the hazard ratio, limiting its value to the range between 0 and 1. Then we have:

$$h(x) = -\frac{1}{\alpha}\log\left(\frac{1}{CSDI(x)} - 1\right) + \frac{\beta}{\alpha}$$

If we denote the logarithm component as

$$c(x) = \log\left(\frac{1}{CSDI(x)} - 1\right)$$

and define $\alpha'$ as $-1/\alpha$ and $\beta'$ as $\beta/\alpha$, we can simplify the equation as follows:

$$h(x) = \alpha'c(x) + \beta'$$

This function serves as the activation function for the CSDI-based risk classification in the present example. The use of CSDI can simplify the mathematical calculations of the model for mapping tile image feature data onto the cancer specific survival time, thereby helping to avoid potential overfitting.

In the optimization part of the neural network model, we use Average Negative Log Partial Likelihood Loss to optimize the model, and its loss function is as follows:

$$L = -\frac{1}{N_{E=1}}\sum_{i:E_i=1}\left(\widehat{h(x_i)} - \log\left(\sum_{j\in R(T_i)}e^{\widehat{h(x_j)}}\right)\right)$$

In this equation:

- $N_{E=1}$ represents the number of patients who had the cancer specific death event.
- $E_i$ is the cancer specific death event representation variable of the i-th patient. (If a death event occurs, this variable is 1, otherwise it is 0.)
- $T_i$ represents the i-th patient's respective time of the cancer specific death event.
- $R(T_i)$ represents the set of patients who have not experienced the cancer specific death event before time $T_i$.
- $x_i$ represents the digital pathology graph feature data of the i-th patient.
- $\hat{h}(x_i)$ is the logarithmic risk value of the i-th patient.

Substituting h(x) with c(x), we have the loss function:

$$L = -\frac{1}{N_{E=1}} \sum_{i:E_i=1} (\alpha' \widehat{c(x_i)} + \beta' - log(\sum_{j \in R(T_i)} e^{\alpha' \widehat{c(x_j)} + \beta'}))$$

The given loss function formula requires that the death events of each batch that is input cannot be empty (since, otherwise, the real part of the logarithm will be 0, and an error will occur during training). To solve this problem, we use the survival event sampling method to ensure that at least one death event occurs in each batch, and balance the data in sequence. The specific sampling operation is as follow: when filling each batch of data, if none of the samples up to the last sample involves a death event, we discard that sample, randomly select one of the death events in the training data, and add it to this batch of data. This helps ensure the smooth progress of the training.

The neural network model for the good prognosis class prediction 520 will now be described in more detail. This model employs the same GIN+CNN architecture as the model 510, but the final output is a binary classification value that indicates whether the patient belongs to the good prognosis group (or not). Because of the imbalance between the good and poor prognosis classes in the training dataset, we employ focal loss (FL) to construct the loss function:

$$\mathrm{FL}(p_t) = -(1 - p_t)^{\gamma} \log(p_t)$$

FL adds a factor $(1 - p_t)^{\gamma}$ to the standard cross entropy criterion. Setting $\gamma > 0$ reduces the relative loss for well-classified examples (pt > 0.5), putting more focus on hard, misclassified examples.

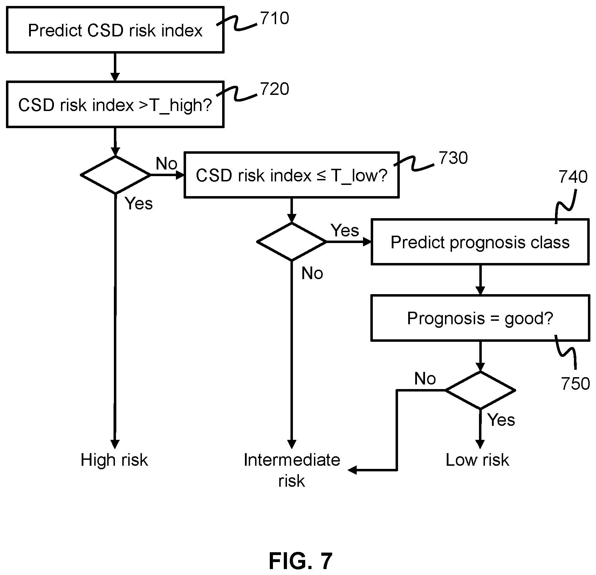

The CSD risk index prediction model 510 and the good prognosis class prediction model 520 form an ensemble learning model for the final prognosis risk prediction. One example of the combination operation 530 of Fig. 5 will now be described, with reference to Fig. 7.

The CSD risk index is predicted in operation 710. This is done using the GIN-based neural network 510 of Fig. 5, as explained above. In operation 720, the computer evaluates whether the predicted risk index is above a threshold $T_{high}$. If so, the risk factor (risk category) is determined to be "high". If the predicted risk index is not above the threshold $T_{high}$, the computer evaluates whether it is below a threshold $T_{low}$. If so, the risk factor (risk category) is determined to be "intermediate". Otherwise, the algorithm proceeds to operation 740, in which the prognosis class is predicted. This is done using the GIN-based neural network 520 of Fig. 5, as explained above. In operation 750, the computer evaluates whether the predicted prognosis class is "good". If so, the risk factor (risk category) is determined to be "low". If not, the risk factor (risk category) is determined to be "intermediate".

The thresholds $T_{high}$ and $T_{low}$ may be determined empirically. Although it has been found to be advantageous, it should be understood that the heuristic algorithm of Fig. 7 represents just one way to combine the output of the two neural networks 510 and 520. Other ways are possible. For example, one or more further neural network layers could be trained to combine the outputs of the two neural networks 510 and 520 in a manner that provides predictions that are in some sense optimal with respect to a training dataset.

The ensemble learning architecture of Figs. 5-7, based on two GIN+CNN models, is believed to enable greater accuracy of the risk class prediction, especially for the patients judged to be at "low" risk. This is a significant benefit, as these patients may be recommended by the system not to receive adjuvant chemotherapy treatment. If a patient is misclassified into the "low risk" group, then they may miss out on treatment that might otherwise have benefitted them.

Patients in the high and intermediate risk groups may be recommended by the system for adjuvant chemotherapy treatment.

The training data for the present implementation was drawn from a dataset of 1176 patients from the international multi-centre study QUASAR 2 (Kerr RS, et al., "Adjuvant capecitabine plus bevacizumab versus capecitabine alone in patients with colorectal cancer (QUASAR 2): an open-label, randomised phase 3 trial", Lancet Oncol. 2016 November; 17(11):1543-1557) and 461 patients from the Cancer Genome Atlas (TCGA) (The Cancer Genome Atlas Network. Comprehensive molecular characterization of human colon and rectal cancer. Nature 487, 330-337, 2012, https://doi.org/10.1038/nature11252). Clinical validation occurred on 3061 patients from the SCOT clinical study (Paul, J., Iveson, T., Midgley, R. et al., "Choice of randomisation time-point in non-inferiority studies of reduced treatment duration: experience from the SCOT study" Trials 12 (Suppl 1), A30, 2011, https://doi.org/10.1186/1745-6215-12-S1-A30).

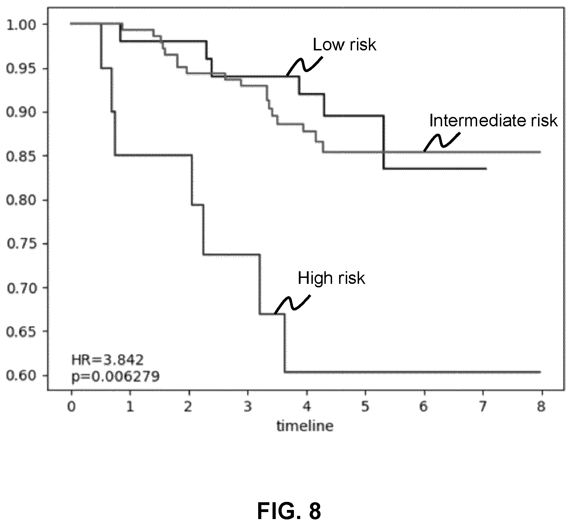

Experimental results show that the proposed algorithm has strong generalization ability and interpretability. A Kaplan-Meier survival analysis on our initial data revealed a 5-year disease-free survival (DFS) of 91%, 85%, and 60% for low, intermediate, and high-risk groups, respectively. These results are shown in the graph of Fig. 8. The X-axis shows the number of years after surgery (resection). The Y-Axis shows the fraction of patients still surviving. The hazard ratio for the high versus low-risk group (HR = 3.842 [95%CI: 1.73-5.03], P=0.0063) was statistically significant, demonstrating the potential of the algorithm to stratify patients based on prognosis risk.

Fig. 9 is a flowchart illustrating a method of training a neural network for use in a method such as the example described above. The training method may be executed by the same computer as the inference method or by a different computer. In the present example, for simplicity, it is assumed that the same computer implements both methods. The initial operations of the method correspond to the operations of the inference method described above with reference to Fig. 1.

In operation 810, the computer obtains a training dataset of histological images. These are of the same type and in the same format as the images obtained in operation 110. In operation 815, the computer obtains patient-outcome data associated with each histological image in the training dataset. The patient outcome data includes an indication of the timing of a cancer specific death (CSD) event (if any) for each patient.

In operation 20, the computer extracts a region of interest from each of the training images. This is done using the same algorithm as described above for operation 120. In operation 830, the region of interest detected in each training image is divided into blocks (in the same manner as operation 130). From the blocks produced by operation 830 for each training image, the computer extracts image features in operation 840. This involves running the same feature extraction process as in operation 140, for each training image. Next, a graph representation is constructed for each training image, in operation 850. This is based on the blocks obtained from operation 830 and the feature data extracted from those blocks in operation 840. The graph construction proceeds in the same manner as operation 150, described previously above.

The neural network is trained in operation 860. The training relies on a backpropagation algorithm.

The computer calculates, for each patient, based on the timing of the CSD event (if any), the logarithmic risk value for that patient. This is used, in the loss function defined above, for training the neural network 510 for CSD risk index prediction.

The computer also determines the risk category for each patient based on the timing of their CSD event (if any). For example, if the patient died within three years (and the death was caused by their cancer), the patient is assigned to the high-risk category. If the patient survived disease-free for more than 5 years, the patient is assigned to the low-risk category. The other patients are assigned to the intermediate-risk category. Note that patients who died within 100 days after resection surgery were excluded from the training dataset, as the death may have been a result of non-natural causes. The assigned risk categories are used for training the neural network 520 for good prognosis class prediction.

In the present example, the two neural networks 510 and 520 are trained concurrently. The DINO-based feature extraction algorithm is also trained concurrently with the two neural networks 510 and 520.

Fig. 10 of the accompanying drawings schematically illustrates an exemplary computer system 900 upon which examples according to the present disclosure may run. The exemplary computer system 900 comprises a computer-readable storage medium 902, a memory 904, a processor 906 and one or more interfaces 908, which are all linked together over one or more communication busses 910. The exemplary computer system 900 may take the form of a conventional computer system, such as, for example, a desktop computer, a personal computer, a laptop, a server, a mainframe computer, and so on.

The computer-readable storage medium 902 and/or the memory 904 may store one or more computer programs (or software or code) and/or data. The computer programs stored in the computer-readable storage medium 902 may include an operating system for the processor 906 to execute in order for the computer system 900 to function. The computer programs stored in the computer-readable storage medium 902 and/or the memory 904 may include computer programs according to examples of the present disclosure or computer programs that, when executed by the processor 906, cause the processor 906 to carry out a method according to an example of the present disclosure.

The processor 906 may be any data processing unit suitable for executing one or more computer readable program instructions, such as those belonging to computer programs stored in the computer-readable storage medium 902 and/or the memory 904. As part of the execution of one or more computer-readable program instructions, the processor 906 may store data to and/or read data from the computer-readable storage medium 902 and/or the memory 904. The processor 906 may comprise a single data processing unit or multiple data processing units operating in parallel or in cooperation with each other. Suitable data processing units may include - but are not limited to - a central processing unit (CPU), a graphics processing unit (GPU), and an artificial intelligence (AI) accelerator, deep learning processor, or neural processing unit (NPU). The computer system 900 may include several data processing units of different types. The processor 906 may, as part of the execution of one or more computer readable program instructions, store data to and/or read data from the computer-readable storage medium 902 and/or the memory 904.

The one or more interfaces 908 may comprise a network interface enabling the computer system 900 to communicate with other computer systems across a network. The network may be any kind of network suitable for transmitting or communicating data from one computer system to another. For example, the network could comprise one or more of a local area network, a wide area network, a metropolitan area network, the internet, a wireless communications network, and so on. The computer system 900 may communicate with other computer systems over the network via any suitable communication mechanism/protocol. The processor 906 may communicate with the network interface via the one or more communication busses 910 to cause the network interface to send data and/or commands to another computer system over the network. Similarly, the one or more communication busses 910 enable the processor 906 to operate on data and/or commands received by the computer system 900 via the network interface from other computer systems over the network. The data received by the computer system 900 may include histological images (including training images for use in a training method according to the present disclosure, and/or images to be analysed using an inference method according to the present disclosure).

The interface 908 may alternatively or additionally comprise a user input interface and/or a user output interface. The user input interface may be arranged to receive input from a user, or operator, of the system 900. The user may provide this input via one or more user input devices (not shown), such as a mouse (or other pointing device, track-ball or keyboard). The user output interface may be arranged to provide a graphical/visual output to a user or operator of the system 900 on a display (or monitor or screen) (not shown). The processor 906 may instruct the user output interface to form an image/video signal which causes the display to show a desired graphical output. The display may be touch-sensitive enabling the user to provide an input by touching or pressing the display.

In some examples according to the present disclosure, the interface 908 may alternatively or additionally comprise an interface to a scanner configured for whole slide imaging. The computer may receive histological images from the scanner via the interface, for processing by the processor 906.

It should be noted that the above-mentioned examples illustrate rather than limit the invention, and that those skilled in the art will be able to design many alternative examples without departing from the scope of the appended claims.

For instance, in the examples described above, it was assumed, for simplicity, that a single histological image is obtained for each patient. This is non-limiting. Two, three or more histological images (for example, two, three, or more whole slide images) may be obtained for a given patient. These may be analysed together or separately using methods according to examples of the present disclosure. The different images may correspond to different microtome sections (slices), for example.

As noted already above, image features may be extracted from all blocks (tiles), or a selected subset of blocks extracted from each histological image. There may be a trade-off between computational complexity and predictive accuracy. However, in general, good performance can be achieved without analysing features from every block.

The examples above have focused on colorectal cancer tumours. The same or similar methods for prognosis prediction may be applied to other solid tumours of other types. The analysis methods may be applied to benign, precancerous, or malignant tumours (or generally to tumours that have not yet been classified into one of these classes).

In the examples discussed above, various neural network architectures were used. In other examples, other neural network architectures may be used. In particular, neural networks other than those based on DeepLabv3+ and/or MobileNetV2 may be used for region of interest detection; and neural networks other than those based on DINO and/or Vision Transformers may be used for feature extraction.

Graph construction may be based on a variety of suitable rules. That is, it is not essential to use the graph construction rules described in the examples above in order to capture an informative representation of a histological image. Nevertheless, the graphs constructed using the rules described above has been found to produce good results in the context of the overall image analysis and risk factor prediction.

When using a convolutional neural network in conjunction with a graph isomorphism network, as described above, the convolution neural network need not have the architecture shown in Fig. 6. This is merely one example. In other examples, the network may have different numbers of convolutional and pooling layers in different sequences. Clinical parameters may or may not be used in the inference. When used they need not be applied as input to the final layer of the CNN - they could be input to any one of the preceding layers.

In the examples of Fig. 1 and Fig. 9, the histological images were segmented automatically to extract the region of interest. This is not essential. In some examples images may be segmented manually - for example, through manual annotation by a pathologist. In other examples, there might be no explicit, separate detection of the region of interest. Instead, the histological image as a whole may be converted to a graph representation and processed by a neural network to determine the risk factor.

A loss function was defined above, for training the neural network 510 for CSD risk index prediction. This has been found to produce good experimental results; however, it should be understood that the scope of the present disclosure is not limited to the use of this loss function. Those skilled in the art will be able to devise suitable alternative loss functions based on this example.

In the claims, any reference signs placed between parentheses shall not be construed as limiting the claim. The word "comprising" does not exclude the presence of elements or operations other than those listed in a claim. The word "a" or "an" preceding an element does not exclude the presence of a plurality of such elements. The examples may be implemented by means of hardware comprising several distinct elements. In a device claim enumerating several means, several of these means may be embodied by one and the same item of hardware. The mere fact that certain measures are recited in mutually different dependent claims does not indicate that a combination of these measures cannot be used to advantage. Furthermore, in the appended claims lists comprising "at least one of: A; B; and C" should be interpreted as (A and/or B) and/or C.

Furthermore, in general, the various examples may be implemented in hardware or special purpose circuits, software, logic or any combination thereof. For example, some aspects may be implemented in hardware, while other aspects may be implemented in firmware or software which may be executed by a controller, microprocessor, or other computing device, although these are not limiting examples. While various aspects described herein may be illustrated and described as block diagrams, flow charts, or using some other pictorial representation, it is well understood that these blocks, apparatus, systems, techniques or methods described herein may be implemented in, as non-limiting examples, hardware, software, firmware, special purpose circuits or logic, general purpose hardware or controller or other computing devices, or some combination thereof.

The examples described herein may be implemented by computer software executable by a data processor of the apparatus, such as in the processor entity, or by hardware, or by a combination of software and hardware. Further in this regard it should be noted that any blocks of the logic flow as in the Figures may represent program operations, or interconnected logic circuits, blocks and functions, or a combination of program operations and logic circuits, blocks, and functions. The software may be stored on such physical media as memory chips, or memory blocks implemented within the processor, magnetic media such as hard disk or floppy disks, and optical media such as for example DVD and the data variants thereof.

The memory may be of any type suitable to the local technical environment and may be implemented using any suitable data storage technology, such as semiconductor-based memory devices, magnetic memory devices and systems, optical memory devices and systems, fixed memory, and removable memory. The data processors may be of any type suitable to the local technical environment, and may include one or more of general-purpose computers, special purpose computers, microprocessors, digital signal processors (DSPs), application specific integrated circuits (ASIC), gate level circuits and processors based on multi-core processor architecture, as non-limiting examples.

Examples as discussed herein may be practiced in various components such as integrated circuit modules. The design of integrated circuits is by and large a highly automated process. Complex and powerful software tools are available for converting a logic level design into a semiconductor circuit design ready to be etched and formed on a semiconductor substrate.

## Claims

1. A computer-implemented method for predicting a risk factor for a patient based on histopathological image analysis, the method comprising:

    receiving (110) at least one histological image showing a sample of a solid tumour;
    converting (150) the histological image into a graph representation;
    processing (160) the graph representation using a neural network, wherein the neural network comprises a graph isomorphism network and a convolutional neural network; and
    determining (162) the risk factor based on an output of the neural network.

2. The method of claim 1, wherein

    the neural network is a first neural network;
    the output of the first neural network comprises a risk index prediction; and
    the method comprises:

        processing the graph representation using a second neural network; and
        determining the risk factor based on the risk index prediction and an output of the second neural network.

3. The method of claim 2, wherein the second neural network comprises a second graph isomorphism network and a second convolutional neural network.

4. The method of claim 2 or claim 3, wherein the output of the second neural network comprises a prognosis class prediction.

5. The method of any one of claims 2 to 4, wherein the risk index prediction is a prediction of a cancer specific death risk index.

6. The method of any one of claims 2 to 5, wherein determining the risk factor comprises combining the risk index prediction with the output of the second neural network.

7. The method of any one of the preceding claims, wherein the neural network receives as an input one or more clinical parameters of the patient, and the output of the neural network is based at least in part on the one or more clinical parameters.

8. The method of any one of the preceding claims, wherein converting the histological image into the graph representation comprises dividing (130) at least a part of the image into blocks of pixels and constructing (150) the graph representation based on the blocks.

9. The method of claim 8, wherein converting the histological image into the graph representation comprises extracting (140) a plurality of features from each of at least some of the blocks, wherein the extracting comprises applying the respective block as input to a neural network configured in a self-distillation with no labels, hereinafter DINO, architecture.

10. The method of any one of the preceding claims, wherein converting the histological image into a graph representation comprises extracting (120) a region of interest from the histological image and constructing (150) the graph representation based on the region of interest.

11. The method of claim 10, wherein extracting the region of interest comprises applying a first machine learning model (310) a first scale and applying a second machine learning model (312) at a second scale.

12. A computer implemented method of training a machine learning architecture for predicting a risk factor for a patient based on histopathological image analysis, the machine learning architecture comprising a neural network, the method comprising:

   obtaining (810) a plurality of histological images;
   obtaining (815) a patient outcome associated with each image;
   converting (850) each histological image into a respective graph representation; and
   training (860) the neural network to predict the risk factor using the graph representations and the respective patient outcomes,
   wherein the neural network comprises a graph isomorphism network and a convolutional neural network.

13. The method of any one of the preceding claims, wherein the patient has a cancer, optionally a colorectal cancer.

14. A computer program comprising computer program code configured to cause one or more physical computing devices to perform a method according to any one of the preceding claims when said computer program code is run on the one or more physical computing devices.

15. A system (900) for predicting a risk factor for a patient based on histopathological image analysis, the system comprising:

   an input (908), for receiving (110) at least one histological image showing a sample of a solid tumour; and
   one or more processors (906), configured to:

      convert (150) the histological image into a graph representation;
      process (160) the graph representation using a neural network, wherein the neural network comprises a graph isomorphism network and a convolutional neural network; and

determine (162) the risk factor based on an output of the neural network.

| Obtain at least one histological image of tumour | 110 |

↓

| Extract region of interest | 120 |

↓

| Divide region of interest into blocks | 130 |

↓

| Extract features from blocks | 140 |

↓

| Construct graph representation | 150 |

↓

| Process graph using neural network | 160 |

↓

| Determine risk factor | 162 |

**FIG. 1**

FIG. 2

ROI Detection
Model 1 — 310

ROI Detection
Model 2 — 312

Mask upscaling — 320

Mask merge — 322

330

Bitwise OR

Dilation and closing — 340

Merge ROI areas — 350

Exclude small areas — 360

**FIG. 3**

$p_1$

softmax — 426

Student encoder — 420

Transformation 1 — 412

$p_2$

softmax — 436

centering — 434

Teacher encoder — 430

Transformation 2 — 414

Block — 401

FIG. 4

510

GIN-based CSD risk index prediction

530

Graph + Block feature data

Prognosis risk category

GIN-based good prognosis class prediction

520

**FIG. 5**

610

GIN

622

Convolutional layer

624

Pooling layer

626

Convolutional layer

628

Pooling layer

Clinical parameters

629

Fully connected layer

CNN

620

**FIG. 6**

```
Predict CSD risk index                  710

          ↓

CSD risk index >T_high?                  720

          ↓
                 No
        ◇ ──────────→  CSD risk index ≤ T_low?        730
        │
        Yes                    ↓
                                      Yes
                              ◇ ──────────→  Predict prognosis class    740
                              │
                              No                    ↓

                                              Prognosis = good?

                                                    ↓
                                          No
                                       ◇ ──────        750
                                          │  Yes

  High risk            Intermediate            Low risk
                          risk
```

**FIG. 7**

FIG. 8

Obtain histological images ⟿ 810

Extract region of interest ⟿ 820

Divide region of interest into blocks ⟿ 830

Extract features from blocks ⟿ 840

Construct graph representation ⟿ 850

Obtain patient outcomes ⟿ 815

860 ⟿ Train neural network

**FIG. 9**

FIG. 10

Europäisches
Patentamt

European
Patent Office

Office européen
des brevets

## EUROPEAN SEARCH REPORT

**Application Number**

EP 25 17 4093

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LU WENQI ET AL: "Capturing Cellular Topology in Multi-Gigapixel Pathology Images", 2020 IEEE/CVF CONFERENCE ON COMPUTER VISION AND PATTERN RECOGNITION WORKSHOPS (CVPRW), IEEE, 14 June 2020 (2020-06-14), pages 1049-1058, XP033798935, DOI: 10.1109/CVPRW50498.2020.00138 * The whole document, in particular: Abstract; Pages 1050 - 1053; Figures 1, 2. * | 1-15 | INV. G16H50/30 G16H50/20 G16H30/40 G16H50/70 G06T7/00 |
| X | WOOD RUBY ET AL: "Joint Prediction of Response to Therapy, Molecular Traits, and Spatial Organisation in Colorectal Cancer Biopsies", 1 October 2023 (2023-10-01), MEDICAL IMAGE COMPUTING AND COMPUTER ASSISTED INTERVENTION - MICCAI 2023; [LECTURE NOTES IN COMPUTER SCIENCE; LECT.NOTES COMPUTER], SPRINGER NATURE SWITZERLAND, CHAM, PAGE(S) 758 - 767, XP047670981, ISSN: 0302-9743 ISBN: 978-3-031-43903-2 [retrieved on 2023-10-01] * The whole document, in particular: Abstract; Pages 760 - 762; Figure 1. * | 1-15 | |

-----

-----

-/--

**TECHNICAL FIELDS SEARCHED (IPC)**

G16H
G06N
G06T

The present search report has been drawn up for all claims

| Place of search | Date of completion of the search | Examiner |
|---|---|---|
| Munich | 8 August 2025 | Nagele, Stefan |

page 1 of 3

**EUROPEAN SEARCH REPORT**

Europäisches
Patentamt
European
Patent Office
Office européen
des brevets

**Application Number**

EP 25 17 4093

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anklin Valentin ET AL: "Learning Whole-Slide Segmentation from Inexact and Incomplete Labels Using Tissue Graphs", Lecture Notes in Computer Science, International Conference on Medical Image Computing and Computer-Assisted Intervention, 21 September 2021 (2021-09-21), XP093216229, DOI: 10.1007/978-3-030-87196-359 Retrieved from the Internet: URL:https://link.springer.com/content/pdf/10.1007/978-3-030-87196-3_59.pdf?pdf=inline%20link [retrieved on 2024-10-18] * The whole document, in particular: Abstract; Pages 638 - 641; Figures 1 - 3. * | 1-15 | |
| X | PATI PUSHPAK ET AL: "HACT-Net: A Hierarchical Cell-to-Tissue Graph Neural Network for Histopathological Image Classification", 5 October 2020 (2020-10-05), 20201005, PAGE(S) 208 - 219, XP047564876, * The whole document, in particular: Abstract; Pages 210 - 213; Figures 1 - 3. * | 1-15 | **TECHNICAL FIELDS SEARCHED (IPC)** |
| A | DAVID AHMEDT-ARISTIZABAL ET AL: "A Survey on Graph-Based Deep Learning for Computational Histopathology", ARXIV.ORG, CORNELL UNIVERSITY LIBRARY, 201 OLIN LIBRARY CORNELL UNIVERSITY ITHACA, NY 14853, 1 July 2021 (2021-07-01), XP091006123, * The whole document, in particular: Abstract. * | 1-15 | |

-/--

The present search report has been drawn up for all claims

| Place of search | Date of completion of the search | Examiner |
|---|---|---|
| Munich | 8 August 2025 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

page 2 of 3

Europäisches
Patentamt

European
Patent Office

Office européen
des brevets

## EUROPEAN SEARCH REPORT

**Application Number**

EP 25 17 4093

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MENG XIANGYAN ET AL: "Clinical applications of graph neural networks in computational histopathology: A review", COMPUTERS IN BIOLOGY AND MEDICINE, NEW YORK, NY, US, vol. 164, 30 June 2023 (2023-06-30), XP087392777, ISSN: 0010-4825, DOI: 10.1016/J.COMPBIOMED.2023.107201 [retrieved on 2023-06-30] * The whole document, in particular: Abstract. *<br><br>----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 8 August 2025 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIANG-CHIEH CHEN** ; **YUKUN ZHU** ; **GEORGE PAPANDREOU** ; **FLORIAN SCHROFF** ; **HARTWIG ADAM**. Encoder-Decoder with Atrous Separable Convolution for Semantic Image Segmentation. *European Conference on Computer Vision*, 2018, 833-851
- **M MACENKO** ; **M NIETHAMMER** ; **JS MARRON** ; **D BORLAND** ; **JT WOOSLEY** ; **G XIAOJUN** ; **C SCHMITT** ; **NE THOMAS**. A method for normalizing histology slides for quantitative analysis. *IEEE ISBI*, 2009
- **MATHILDE CARON** ; **HUGO TOUVRON** ; **ISHAN MISRA** ; **HERVÉ JÉGOU** ; **JULIEN MAIRAL** ; **PIOTR BOJANOWSKI** ; **ARMAND JOULIN**. *Emerging Properties in Self-Supervised Vision Transformers*, May 2021, https://arxiv.org/abs/2104.14294.

- **KERR RS et al.** Adjuvant capecitabine plus bevacizumab versus capecitabine alone in patients with colorectal cancer (QUASAR 2): an open-label, randomised phase 3 trial. *Lancet Oncol.*, November 2016, vol. 17 (11), 1543-1557
- The Cancer Genome Atlas Network. Comprehensive molecular characterization of human colon and rectal cancer. *Nature*, 2012, vol. 487, 330-337, https://doi.org/10.1038/nature11252
- **PAUL, J.** ; **IVESON, T.** ; **MIDGLEY, R. et al.** Choice of randomisation time-point in non-inferiority studies of reduced treatment duration: experience from the SCOT study. *Trials*, 2011, vol. 12 (1), A30, https://doi.org/10.1186/1745-6215-12-S1-A30